**Europäisches Patentamt**

**European Patent Office**

**Office européen des brevets**

(19)

(11)  **EP 1 120 395 A2**

(12)  **EUROPÄISCHE PATENTANMELDUNG**

(43) Veröffentlichungstag:
**01.08.2001  Patentblatt 2001/31**

(51) Int Cl.$^7$: **C07C 69/54**, C09D 4/06

(21) Anmeldenummer: **01100789.5**

(22) Anmeldetag: **13.01.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **26.01.2000  DE 10003118**

(71) Anmelder: **Clariant GmbH
65929 Frankfurt am Main (DE)**

(72) Erfinder:
• **Heinrichs, Franz-Leo, Dr.
86456 Gablingen (DE)**
• **Krendlinger, Ernst, Dr.
86316 Friedberg (DE)**
• **Ehrhardt, Heinz, Dr.
86508 Rehling (DE)**
• **Henze, Andree, Dr.
86199 Augsburg (DE)**

(54)  **Polymerisierbare Verbindungen und ihre Verwendung als Wachs**

(57)  Gegenstand der Erfindung sind Verbindungen der Formel

$$Z_k - (Y - (W)_{(m-p-1-k)}(X)_q)_n$$

worin

Z  der Rest ist, welcher durch Entfernung der OH-Gruppen aus COOH-Gruppen einer organischen Carbonsäure, welche 1 - 4 COOH-Gruppen enthält und deren Anzahl von kohlenstoffatomen zwischen 4 und 40 liegt, erhalten wurde,

Y  der Rest ist, welcher durch Entfernung von (m-p) Wasserstoffatomen aus den Hydroxylgruppen einer organischen, m OH-Gruppen enthaltenden Verbindung mit 3 bis 15 Kohlenstoffatomen erhalten wurde, wobei Y ein mit $C_2$-$C_4$-Alkylenoxiden alkoxylierter Rest sein kann, und von der Gesamtzahl m der OH-Gruppen (m-p) OH-Gruppen verestert sind, und p OH-Gruppen frei oder verethert vorliegen,

X  der einwertige Rest ist, der durch Entfernung der OH-Gruppe aus der COOH-Gruppe einer organischen Monocarbonsäure mit 3 - 5 C-Atomen welche eine, in Konjugation mit der C-O-Doppelbindung stehende, olefinische Doppelbindung enthält, erhalten wurde,

W  der einwertige Rest ist, der durch Entfernung der OH-Gruppe aus der COOH-Gruppe einer einwertigen Carbonsäure mit 16 - 60 Kohlenstoffatomen erhalten wurde,

p  eine Zahl von 0 bis 2,5, die die Anzahl der freien oder veretherten OH-Gruppen angibt,

k  eine Zahl von 0 bis 2

q  eine Zahl von 0,5 bis 1,5, die die Anzahl der Estereinheiten der ungesättigten Säure X angibt,

n  eine ganze Zahl von 1 bis 6 unter der Bedingung, dass k > 0 ist, wobei n dann die Anzahl der Wiederholungseinheiten der erfindungsgemäßen Verbindung angibt,

m  eine ganze Zahl von 3 bis 8 ist und die Anzahl der OH-Gruppen im mehrwertigen Alkohol angibt,

mit der Maßgabe, dass

$$1 \leqq n \cdot q \leqq 6,$$

$$2 \leqq (n[m-p-1]) \leqq 15,$$

$$m - p - 1 > 0,$$

und

$$m - n - 1 - k > 1$$

ist.

**EP 1 120 395 A2**

**Beschreibung**

Die vorliegende Erfindung betrifft ethylenisch ungesättigte Verbindungen mit langen Alkylketten, sowie deren Verwendung als copolymerisierbarer Wachsbestandteil für Oberflächenbeschichtungen.

Anwendungsbereiche für wachsartige Substanzen sind beispielsweise die Gebiete Hydrophobieren, Mattieren, Dispergieren, Trennen, Weichmachen, Viskositätsregelung, Coaten, Glätten von Oberflächenstrukturen, Emulgieren, Schmelzpunkteinstellung, Gleiten, Retardieren, Glanzeinstellung, Flexibilisierung, Oberflächenschutz, Bindemittel und Verträglichkeitsvermittlung.

Bei den meisten Anwendungen wird das Wachs als Additiv eingesetzt und verbleibt im Endprodukt. Gute Verträglichkeit und hohe Molgewichte unterstützen diesen Verbleib in einer Substratmatrix, aber zunehmend wird für Substrate/Produkte beispielsweise in Kontakt mit Lebensmitteln gefordert, daß keine Bestandteile an die Umgebung abgegeben werden dürfen, weder durch Ausschwitzen noch durch Extraktion. Das gilt sowohl für die Basismaterialien als auch für die Additive, wie beispielsweise Wachse.

Im Bereich der UV-härtenden Beschichtungen, insbesondere bei Lacken und Druckfarben, wurde durch Optimierung des Härtungsprozesses der Anteil an extrahierbaren Substanzen, hauptsächlich Monomere und Reaktiwerdünner, gesenkt.

Als Additive zur Verbesserung von Viskosität, Fließverhalten und Klebrigkeit werden in DE-A-24 23 354 Partialester mehrwertiger Alkohole beschrieben, in denen mehrere Acrylestergruppen enthalten sind und die somit in den Film netzwerkartig einpolymerisiert werden können. Es handelt sich dabei um Produkte, die bestimmungsgemäß mit dem Bindemittelsystem gut verträglich sind. Sie haben jedoch zu niedrige Schmelzpunkte.

Bei Additiven zur Einstellung von Scheuerschutz, Gleitreibung, Oberflächengriff, Glanz und Mattierung , wie z.B. mikronisierte Wachse , die als Partikel im Film vorliegen, ist das Problem der dauerhaften Einbindung der Partikel in den Film noch nicht gelöst. Sowohl aus der Oberfläche als auch aus dem Film kann das Wachs durch Extraktion eluiert werden.

Aufgrund dieses Problems bestand die vorliegender Erfindung zugrunde liegende Aufgabe darin, Wachse zu finden, die in der Oberflächenbeschichtung durch gesteigerte Zurückhaltung im Beschichtungsmaterial ausgezeichnet sind. Darüber hinaus sollen die Wachse eine hinreichende mechanische Festigkeit für Oberflächenbeschichtungen aufweisen.

Überraschenderweise wurde gefunden, dass reaktiv einbindbare wachsartige Verbindungen diese Aufgabe lösen, wenn sie begrenzte Anteile an polymerisierbaren Doppelbindungen aufweisen.

Gegenstand der Erfindung sind daher Verbindungen der Formel

$$Z_k - (Y - (W)_{(m-p-1-k)}(X)_q)_n$$

worin

Z　der Rest ist, welcher durch Entfernung der OH-Gruppen aus COOH-Gruppen einer organischen Carbonsäure, welche 1 - 4 COOH-Gruppen enthält und deren Anzahl von Kohlenstoffatomen zwischen 4 und 40 liegt, erhalten wurde,

Y　der Rest ist, welcher durch Entfernung von (m-p) Wasserstoffatomen aus den Hydroxylgruppen einer organischen, m OH-Gruppen enthaltenden Verbindung mit 3 bis 15 Kohlenstoffatomen erhalten wurde, wobei Y ein mit $C_2$-$C_4$-Alkylenoxiden alkoxylierter Rest sein kann, und von der Gesamtzahl m der OH-Gruppen (m-p) OH-Gruppen verestert sind, und p OH-Gruppen frei oder verethert vorliegen,

X　der einwertige Rest ist, der durch Entfernung der OH-Gruppe aus der COOH-Gruppe einer organischen Monocarbonsäure mit 3 - 5 C-Atomen welche eine, in Konjugation mit der C-O-Doppelbindung stehende, olefinische Doppelbindung enthält, erhalten wurde,

W　der einwertige Rest ist, der durch Entfernung der OH-Gruppe aus der COOH-Gruppe einer einwertigen Carbonsäure mit 16 - 60 Kohlenstoffatomen erhalten wurde,

p　eine Zahl von 0 bis 2,5, die die Anzahl der freien oder veretherten OH-Gruppen angibt,

k　eine Zahl von 0 bis 2

q　eine Zahl von 0,5 bis 1,5, die die Anzahl der Estereinheiten der ungesättigten Säure X angibt,

n　eine ganze Zahl von 1 bis 6 unter der Bedingung, dass k > 0 ist, wobei n dann die Anzahl der Wiederholungseinheiten der erfindungsgemäßen Verbindung angibt,

m　eine ganze Zahl von 3 bis 8 ist und die Anzahl der OH-Gruppen im mehrwertigen Alkohol angibt,

mit der Maßgabe, dass

$$1 \leqq n \cdot q \leqq 6,$$

$$2 \leqq (n[m-p-1]) \leqq 15,$$

$$m - p - 1 > 0,$$

und

$$m - n - 1 - k > 1$$

ist.

Die unter W beschriebene Monocarbonsäure muß hydrophoben Charakter haben. Es müssen daher Carbonsäuren mit $C_{16}$-$C_{60}$, vorzugsweise $C_{22}$-$C_{40}$ als reine Verbindung oder als Säuregemisch verwendet werden. Für die erwünschten Produkteigenschaften ist es wichtig, daß der Hauptanteil der Säure gesättigte, lineare Kohlenwasserstoffketten umfaßt. In untergeordnetem Maß bis 10 mol-% können auch ungesättigte und verzweigte Carbonsäuren in einer solchen Mischung enthalten sein. Geeignete Verbindungen sind beispielsweise Talgfettsäure, Montanwachssäure, Paraffinoxidate, Wachsoxidate, Olefinoxidate, technische $C_{36}$ Guerbetsäure, Behensäure und Erucasäure.

Zur Erreichung des gewünschten Molgewichtes und der Polarität können gegebenenfalls die unter Z beschriebenen mehrwertigen Carbonsäuren verwendet werden, beispielsweise gehärtete Dimerfettsäuren, gehärtete Trimerfettsäure, Dodecandisäure, Adipinsäure, Alkylbernsteinsäure oder Alkenylbernsteinsäure.

Die unter Y beschriebenen mehrwertigen Alkohole sind mindestens dreiwertige Alkohole und/oder deren Ethoxylierungsprodukte, wie beispielsweise Glycerin, Trimethylolpropan, Pentaerythrit, und Sorbitol. Geeignet sind auch die Dimeren und Trimeren der genannten Alkohole, wie z. B. Diglycerin, sofern sie nicht mehr als 8 OH-Gruppen und nicht mehr als 15 C-Atome umfassen. Ist Y alkoxyliert, so trägt es 0 bis 20 Alkoxygruppen, vorzugsweise Ethoxy- oder Propoxygruppen. Die Zahl von 3 bis 15 Kohlenstoffatomen, die der mehrwertige Alkohol Y umfaßt, versteht sich ausschließlich der eventuell vorhandenen Alkoxygruppen.

Der in der Formel mit X gekennzeichnete Rest einer ethylenisch ungesättigten Säure stammt vorzugsweise aus Acrylsäure, Methacrylsäure, oder deren Estern.

Die Gesamtanzahl der Estereinheiten der ethylenisch ungesättigten Säure X, $n \cdot q$, liegt vorzugsweise zwischen 1 und 4.

Die Herstellung der erfindungsgemäßen Verbindungen erfolgt im allgemeinen in einem zweistufigen Verfahren.

In der ersten Stufe wird aus dem mehrwertigen Alkohol Y, der langkettigen Carbonsäure W und gegebenenfalls einer Dicarbonsäure Z der Vorester hergestellt. Die Umsetzung erfolgt ohne Lösemittel mit Standardveresterungskatalysatoren wie Methansulfonsäure, sauren Ionentauschern, Sn-Verbindungen, Titansäureestern oder Na-Methylat. Nach Einstellung der geforderten Säurezahl (SZ) wird dann in einer zweiten Stufe ohne Aufarbeitung die ethylenisch ungesättigten Säure X durch direkte Veresterung oder Umesterung eingebaut. Für die zweite Stufe kann gegebenenfalls der Katalysator gewechselt werden. Die Stöchiometrie für den Einbau der ungesättigten Säure wird so gewählt, daß mindestens 1 mol ungesättigte Säure eingebaut wird, höchstens jedoch 6 mol. Höhere Anteile an Doppelbindungen reduzieren die Härte des Produktes.

Die erfindungsgemäßen Verbindungen sind bei Raumtemperatur feste, wachsartige Verbindungen, die als Feststoffe in micronisierter Form, Schmelze oder Dispersionsform wie bekannte Wachse eingesetzt werden können.

Wachs ist eine technologische Sammelbezeichnung für eine Reihe natürlicher oder künstlich gewonnener Stoffe, die im allgemeinen folgende Eigenschaften aufweisen: bei 20°C knetbar, fest bis brüchig hart, grob- bis feinkristallin, durchscheinend bis opak, nicht glasartig, über 40 °C ohne Zersetzung schmelzend, wenig oberhalb des Schmelzpunktes verhältnismäßig niedrigviskos, stark temperaturabhängige Konsistenz und Löslichkeit, unter leichtem Druck polierbar.

Beispiele

Beispiel 1

| Trimethylolpropan | 1,0 mol |
|---|---|
| Montansäure | 1,5 mol ($C_{28}$-Carbonsäure) |
| Acrylsäure | 1,1 mol |
| Methansulfonsäure 70 %ig | 0,15 Gew.-% |

Die Montansäure wird aufgeschmolzen, Trimethylolpropan und Methansulfonsäure zugesetzt. Dann wird bei 120°C verestert bis eine SZ < 10 erreicht ist. Die Acrylsäure wird zugesetzt und verestert, bis eine SZ < 10 erreicht ist. Der Katalysator wird mit wäßrigem Alkali neutralisiert, Reste flüchtiger Bestandteile werden im Vakuum entfernt. Der Ansatz wird filtriert und konfektioniert. Das erhaltene Produkt zeigt folgende Eigenschaften:

| SZ | 8,5 |
|---|---|
| VZ | 61 |
| Tp. | 78°C |
| SV 90 | 70 mPas |

Beispiel 2

| Trimethylolpropan | 1,0 mol |
|---|---|
| Montansäure | 1,1 mol |
| Adipinsäure | 0,2 mol |
| Acrylsäure | 1,1 mol |
| Methansulfonsäure 70 %ig | 0,15 Gew.-% |

Die Montansäure wird aufgeschmolzen, Trimethylolpropan und Methansulfonsäure zugesetzt. Dann wird bei 120°C verestert bis eine SZ < 10 erreicht ist. Nacheinander werden Adipinsäure und Acrylsäure zugesetzt und verestert, bis eine SZ < 10 erreicht ist. Der Katalysator wird mit wäßrigem Alkali neutralisiert, Reste flüchtiger Bestandteile werden im Vakuum entfernt. Der Ansatz wird filtriert und konfektioniert. Das erhaltene Produkt hat folgende Eigenschaften:

| SZ | 9,5 |
|---|---|
| VZ | 169 |
| Tp. | 77°C |
| SV 90 | 350 mPas |

Beispiel 3

| Pentaerythrit | 1,0 mol |
|---|---|
| Stearinsäure | 2,5 mol |
| Acrylsäuremethylester | 1,1 mol |
| Sn-Katalysator | 0,15 Gew.-% |

Die Stearinsäure wird aufgeschmolzen, Pentaerythrit und Katalysator zugesetzt, dann wird aufgeheizt und bei 190°C verestert bis eine SZ < 10 erreicht ist. Der Ansatz wird abgekühlt auf 120°C und Acrylsäuremethylester zugesetzt und umgeestert, bis kein Methanol mehr abdestilliert. Der Katalysator wird gefällt, Reste flüchtiger Bestandteile werden im Vakuum entfernt, dann wird filtriert und konfektioniert. Das erhaltene Produkt hat folgende Eigenschaften:

| SZ | 9,5 |
|---|---|
| VZ | 194 |
| Tp. | 55°C |
| SV 90 | 50 mPas |

Beispiel 4

| Pentaerythrit | 1,0 mol |
|---|---|
| Stearinsäure | 0,5 mol |
| Montansäure | 2,0 mol |
| Acrylsäure | 1,1 mol |
| Sn-Katalysator | 0,15 Gew.-% |
| Methansulfonsäure 70%ig | 0,1 Gew.-% auf Ansatz |

Montansäure und technische Stearinsäure werden aufgeschmolzen, Pentaerythrit und Katalysator zugesetzt. Dann wird aufgeheizt und bei 190°C verestert bis eine SZ < 10 erreicht ist. Der Ansatz wird abgekühlt auf 120°C, Methansulfonsäure zugesetzt, Acrylsäure zudosiert und verestert bis eine SZ < 10 erreicht ist. Der Katalysator wird neutralisiert, flüchtige Bestandteile im Vakuum abdestilliert, filtriert und konfektioniert. Das erhaltene Produkt hat folgende Eigenschaften:

| SZ | 9,5 |
|---|---|
| VZ | 175 |
| Tp. | 68°C |
| SV 90 | 150 mPas |

Beispiel 5

| Pentaerythrit | 1,0 mol |
|---|---|
| Montansäure | 2,5 mol |
| Acrylsäure | 1,1 mol |
| Methansulfonsäure 70 %ig | 0,15 Gew.-% |

Die Montansäure wird aufgeschmolzen, Trimethylolpropan und Methansulfonsäure zugesetzt. Dann wird bei 120°C verestert bis eine SZ < 10 erreicht ist. Dann wird Acrylsäure zugesetzt und verestert, bis eine SZ < 10 erreicht ist. Der Katalysator wird mit wäßrigem Alkali neutralisiert, Reste flüchtiger Bestandteile werden im Vakuum entfernt. Dann wird filtriert und konfektioniert. Das erhaltene Produkt hat folgende Eigenschaften:

| SZ | 13 |
|---|---|
| VZ | 172 |
| Tp. | 76°C |
| SV 100 | 223 mPas |

In allen Verbindungen gemäß Beispiel 1 - 5 kann der Einbau der Acrylsäure durch 13-C-NMR bestätigt werden.

Anwendungsbeispiele:

Beispiel 6

| UV-härtender Lack: | |
|---|---|
| Laromer® PO 84 F (BASF) | 92,7 % |
| Irgacure® 500 (Ciba) | 3,0 % |
| Tego Glide® 435 (Tego-Chemie) | 0,3 % |
| Wachs gemäß Beispiel 5 | 4,0 % |

Laromer PO 84 F: Ungesättigte Acrylatharze und reaktive Verdünner für die Strahlungshärtung für UV- und elektronenstrahlhärtbare Lacke und Druckfarben auf verschiedenen Untergründen.
Irgacure 500: Eutektische Mischung aus Irgacure 184 und Benzophenon Tego Glide 435: Für wässrige und UV-Systeme empfohlener Zusatz: 0,5 - 1 % zur besseren Substratbenetzung, Verlauf und Gleitfähigkeit

Beispiel 7

| Beschichtung von Papier und Holzoberflächen | |
|---|---|
| Papier | 135 g/m$^2$ |
| Auftrag | 20 g/m$^2$ |
| Einbrenngeschwindigkeit | 50 m/min |
| Leistung | 200 W/cm$^2$ |
| Abstand Lampe-Substrat | 100 mm |

| Beschichtung (micronisiert) | Glanz | Gleitreibung | Rückstand aus Toluol-Extrakt, ppm |
|---|---|---|---|
| Lack ohne Zusatz | 89 % | 0,35 | nicht nachweisbar |
| Muster gemäß Beispiel 5 | 88 % | 0,26 | nicht nachweisbar |
| PE/Amidwachs | 55 % | 0,27 | 250 |
| Amidwachs | 79 % | 0,295 | 350 |
| PE/PTFE | 74 % | 0,265 | 180 |
| PE-Wachs | 72 % | 0,245 | 120 |

Verwendete Abkürzungen:

| VZ | Verseifungszahl |
|---|---|
| Tp. | Tropfpunkt |
| SV | Schmelzviskosität bei der angegebenen Temperatur |
| PE | Polyethylenwachs PE130 |
| Amidwachs | Bis-stearoyl/palmitoylethylendiamin, Wachs C |

**Patentansprüche**

**1.** Verbindungen der Formel

$$Z_k - (Y - (W)_{(m-p-1-k)}(X)_q)_n$$

worin

Z    der Rest ist, welcher durch Entfernung der OH-Gruppen aus COOH-Gruppen einer organischen Carbonsäure, welche 1 - 4 COOH-Gruppen enthält und deren Anzahl von Kohlenstoffatomen zwischen 4 und 40 liegt, erhalten wurde,

Y    der Rest ist, welcher durch Entfernung von (m-p) Wasserstoffatomen aus den Hydroxylgruppen einer organischen, m OH-Gruppen enthaltenden Verbindung mit 3 bis 15 Kohlenstoffatomen erhalten wurde, wobei Y ein mit $C_2$-$C_4$-Alkylenoxiden alkoxylierter Rest sein kann, und von der Gesamtzahl m der OH-Gruppen (m-p) OH-Gruppen verestert sind, und p OH-Gruppen frei oder verethert vorliegen,

X    der einwertige Rest ist, der durch Entfernung der OH-Gruppe aus der COOH-Gruppe einer organischen Monocarbonsäure mit 3 - 5 C-Atomen welche eine, in Konjugation mit der C-O-Doppelbindung stehende, olefinische Doppelbindung enthält, erhalten wurde,

W    der einwertige Rest ist, der durch Entfernung der OH-Gruppe aus der COOH-Gruppe einer einwertigen Carbonsäure mit 16 - 60 Kohlenstoffatomen erhalten wurde,

p    eine Zahl von 0 bis 2,5, die die Anzahl der freien oder veretherten OH-Gruppen angibt,

k    eine Zahl von 0 bis 2

q    eine Zahl von 0,5 bis 1,5, die die Anzahl der Estereinheiten der ungesättigten Säure X angibt,

n    eine ganze Zahl von 1 bis 6 unter der Bedingung, dass k > 0 ist, wobei n dann die Anzahl der Wiederholungseinheiten der erfindungsgemäßen Verbindung angibt,

m    eine ganze Zahl von 3 bis 8 ist und die Anzahl der OH-Gruppen im mehrwertigen Alkohol angibt,

mit der Maßgabe, dass

$$1 \leqq n \cdot q \leqq 6,$$

$$2 \leqq (n[m-p-1]) \leqq 15,$$

$$m - p - 1 > 0,$$

und

$$m - n - 1 - k > 1$$

ist.

2.    Verbindungen nach Anspruch 1, gekennzeichnet durch die Verwendung von Monocarbonsäuren W mit 22 bis 40 C-Atomen.

3.    Verbindungen gemäß Anspruch 1 und/oder 2, gekennzeichnet durch die Verwendung von Acrylsäure, Methacrylsäure oder deren Ester als ethylenisch ungesättigte Säure X.

4.    Verwendung von Verbindungen gemäß einem oder mehreren der Ansprüche 1 bis 3 als copolymerisierbarer, wachsartiger Zusatz zu Oberflächenbeschichtungen.